Europäisches Patentamt

European Patent Office

Office européen des brevets

(11) Veröffentlichungsnummer: **0 017 725**
**B1**

(19)

(12) # EUROPÄISCHE PATENTSCHRIFT

(45) Veröffentlichungstag der Patentschrift:
04.11.81

(51) Int. Cl.³: **B 01 J 23/66, C 07 D 301/10**

(21) Anmeldenummer: 80100796.4

(22) Anmeldetag: 18.02.80

(54) Verfahren zur Herstellung von Silberkatalysatoren für die Herstellung von Ethylenoxid.

(30) Priorität: 11.04.79 DE 2914640

(43) Veröffentlichungstag der Anmeldung:
29.10.80 Patentblatt 80/22

(45) Bekanntmachung des Hinweises auf die Patenterteilung:
04.11.81 Patentblatt 81/44

(84) Benannte Vertragsstaaten:
**BE GB IT NL**

(56) Entgegenhaltungen:
**US-A-2 446 132**
**US-A-2 671 764**
**US-A-4 010 115**

(73) Patentinhaber: CHEMISCHE WERKE HÜLS AG,
Postfach 1320, D-4370 Marl 1 (DE)

(72) Erfinder: Vangermain, Erwin, Dr., Leverkusener Strasse 1,
D-4370 Marl (DE)
Erfinder: Brauckmann, Willi, Sachsenstrasse 6,
D-4370 Marl (DE)
Erfinder: Erberich, Hans-Jürgen, Dr., Leverkusener
Strasse 12, D-4370 Marl (DE)
Erfinder: Ueberschaer, Horst, Bitterfelder Strasse 2,
D-4370 Marl (DE)

## Verfahren zur Herstellung von Silberkatalysatoren für die Herstellung von Ethylenoxid

Die Herstellung von Ethylenoxid durch Oxidation von Ethylen wird bekanntlich in Gegenwart silberhaltiger Trägerkatalysatoren vorgenommen. Die Katalysatoren für die Oxidation des Ethylens zu Ethylenoxid werden im wesentlichen nach folgenden Verfahren hergestellt, nämlich

a) durch Aufbringen der aktiven Silberschicht mit Hilfe einer Suspension auf einen Träger (Suspensionsverfahren); wobei mechanische Misch- oder Sprühtechniken benutzt werden;

b) durch Imprägnieren eines porösen Trägermaterials mit Hilfe z. B. einer Silbersalzlösung (Imprägnierverfahren); die Katalysatoren werden einer thermischen Nachbehandlung unterworfen.

Bei dem sogenannten »Suspensionsverfahren« wird üblicherweise eine wäßrige Aufschlämmung von Silberoxid auf ein keramisches Trägermaterial aufgezogen, wobei eine mehr oder weniger dicke, zusammenhängende zu aktivierende Silberschicht auf dem Träger ausgebildet wird. Diese Silberschicht kann leicht mechanisch beschädigt werden, wodurch sich über das Reaktionsrohr durch anfallenden Silberstaub eine erhöhte Druckdifferenz ausbildet. Dieser führt dann zu einer Verringerung des Gasdurchsatzes und damit zu einer Verminderung der Leistungsfähigkeit des Reaktors. Um diesen Nachteil zu vermeiden, bemüht man sich zu erreichen, daß die Silberoxidpartikel möglichst tief in die Trägerporen eindringen, um diese so gegen mechanischen Abrieb zu schützen und so die Lebensdauer des Katalysators zu verlängern.

Bei dem sogenannten »Imprägnierverfahren« wird anstelle einer Suspension von Silberoxid eine wäßrige Lösung eines Silbersalzes eingesetzt und der Träger in diese Lösung eingetaucht. Nach dem Aufziehen der Silberverbindung in die Poren des Trägers wird der imprägnierte Träger getrocknet und thermisch nachbehandelt. Der besondere Nachteil dieser Katalysatoren liegt darin, daß sie zwar eine gute Selektivität, aber nur einen geringen Umsatz ermöglichen

Es ist auch bekannt, den Katalysator Modifizierungsmittel (Promotoren) zuzusetzen. Als Promotoren werden vor allem Verbindungen der Erdalkali-, Erd- und Seltenerdmetalle empfohlen. Auch der Zusatz von Alkalimetallen und, insbesondere der »schweren« Alkalimetalle, Kalium, Rubidium und Cäsium, ist bekannt (DE-A-2 300 512, DE-A-2 733 688). Es liegen widersprüchliche Aussagen über das optimale Herstellen solcher Katalysatoren vor.

Je nachdem, ob man diese Promotoren auf den Träger in einem vorgeschalteten Verfahrensschritt, während des Aufbringens der Silberverbindung oder nach beendeter Herstellung des primären Silberkatalysators einarbeitet, werden widersprüchliche Ergebnisse hinsichtlich Selektivität und Umsetzungsgrade bei gegebener Reaktionstemperatur beschrieben.

Ein besonders vorteilhaftes Verfahren zur Herstellung von Silberkatalysatoren für das Herstellen von Ethylenoxid, ist ein im folgenden als »gemischtes Verfahren« bezeichnetes, welches in der DE-B-2 017 733 beschrieben ist. Hierbei wird eine wäßrige Silberoxidsuspension eingesetzt, in welcher ein bestimmter Teil des Silbers als Silbersalz vorliegt. Nach diesem Verfahren werden sowohl die Vorteile des zuvor genannten Imprägnierverfahrens als auch die des Suspensionsverfahrens erreicht, ohne daß die jeweiligen Nachteile auftreten.

Ausgehend von diesem Verfahren wurde nun gefunden, daß man auch die an sich bekannten Promotoren vorteilhaft anwenden kann, wenn man bei der Herstellung bestimmte Maßnahmen einhält wie sie in den Ansprüchen beschrieben sind. Insbesondere werden damit Katalysatoren erhalten, welche besonders aktiv sind und nicht nur hohe Selektivität, sondern auch eine hohe Raum-Zeit-Ausbeute erlauben.

Die Katalysatoren werden zweckmäßigerweise wie folgt hergestellt:
Zunächst wird in bekannter Weise aus einer wäßrigen Lösung eines Silbersalzes, üblicherweise Silbernitrat, mit einer wäßrigen Lösung von Natriumhydroxid oder Kaliumhydroxid Silberoxid gefällt. Das abfiltrierte Silberoxid wird so lange mit destilliertem Wasser gewaschen, bis das Waschwasser salzfrei abläuft. 35 bis 65 Mol.-%, vorteilhaft 45 bis 55 Mol.-%, insbesondere 50 Mol.-% des Silberoxids, werden mit Hilfe einer organischen Säure in bekannter Weise zum löslichen Silbersalz umgesetzt.

Die so erhaltene Mischung wird nun mit dem Trägermaterial in ein heiz- und drehbares Mischgefäß gegeben. Bei Temperaturen zwischen 20 und 100°C wird zweckmäßig unter vermindertem Druck das wäßrige Silberoxid-Silbersalzgemisch auf und in die Poren des Trägers gebracht, wobei das angelegte Vakuum nicht konstant gehalten, sondern durch kurzzeitiges Belüften der Druck erhöht wird, jedoch nicht bis auf vollen Atmosphärendruck. Wie oft der Druckwechsel vorgenommen wird, richtet sich nach den vorliegenden Gegebenheiten. Im allgemeinen wird man im Verlauf von einer Minute den Druckwechsel vornehmen.

Zur teilweisen Umwandlung des primär gebildeten Silberoxids in ein wasserlösliches Silbersalz werden einbasige, zweibasige oder dreibasige organische Säuren eingesetzt. Das im Silbersalz vorliegende Anion ist dabei nicht kritisch, sollte jedoch keine Katalysatorgifte bilden. Verwendung finden hierbei organische Carbonsäuren wie Ameisensäure, Essigsäure, Oxalsäure, Malonsäure, Glutarsäure; als Hydroxicarbonsäuren finden Anwendung: Milchsäure, Zitronensäure, Weinsäure oder Glykolsäure. Vorteilhaft verwendet man als Silbersalz Silberlactat und als organische Carbonsäure entsprechend die Milchsäure.

Der feuchte Katalysator wird einer Zwischentrocknung unterworfen. Dabei ist wesentlich, daß das Trocknen in einer dünnen bewegten Schicht unter einer Inertgasatmosphäre vorgenommen wird, wobei das Inertgas zweckmäßigerweise im Gegenstrom geführt wird. Als Inertgas wird vorteilhaft Stickstoff verwendet. Die Temperatur liegt im Bereich von 50 bis 150°C, vorteilhaft bei 120°C, insbesondere bei 110°C. Die Temperaturdifferenz innerhalb des Bandes kann bis zu 10°C betragen. Unter dünner Schicht wird eine Schichthöhe verstanden, die nicht wesentlich mehr als zweimal den Korndurchmesser des Katalysators beträgt. Als besonders vorteilhaft haben sich Siebbandmaschinen erwiesen, die mit einem V4A-Drahtgeflecht ausgerüstet sind, wobei das Band von oben und unten beheizt werden kann. Die Verweilzeit beträgt im allgemeinen 10 bis 720 Minuten, insbesondere 30 bis 300 Minuten. Die Verweilzeit kann durch die Bandgeschwindigkeit reguliert werden.

In einem nächsten Schritt wird eine weitere Silberschicht auf den Träger aufgebracht, wobei ebenfalls eine wäßrige Silberoxid-Silbersalz-Suspension unter den zuvor genannten Bedingungen aufgebracht wird. In dieser Stufe werden jedoch der Suspension Barium- und Rubidium- und/oder Cäsiumverbindungen zugesetzt. Die Verbindungen dieser Promotormetalle liegen vorteilhaft als wasserlösliche Verbindungen vor. Insbesondere sind geeignet, Bariumperoxid, Rubidium- bzw. Cäsiumnitrat bzw. die Hydroxide, insbesondere bieten sich auch die milchsauren Salze an. Selbstverständlich sollen die Verbindungen keine Anteile enthalten, die als Katalysatorgriffe bekannt sind, wie Schwefelverbindungen, Eisenoxide und Quecksilberverbindungen sowie Chlorverbindungen.

Die Menge der zugesetzten Verbindungen wird so bemessen, daß im fertigen Katalysator als Metall gerechnet 0,01 bis 0,25 Gew.-%, vorzugsweise 0,02 insbesondere 0,05 Gew.-% Barium und 0,001 bis 0,05 Gew.-%, vorzugsweise 0,007—0,015, insbesondere 0,01 Gew.-% Rubidium und/oder Cäsium vorliegen.

Anschließend wird der so behandelte Katalysator wiederum in einer dünnen Schicht getrocknet oder unmittelbar in feuchter Form ebenfalls in dünner bewegter Schicht aktiviert. Zweckmäßigerweise geschieht dies in einer weiteren Siebbandmaschine, die in gleicher Weise mit Dampfregistern ausgerüstet ist. Das Aktivieren geschieht bei 160 bis 270°C, vorzugsweise bei 250, insbesondere bei 240°C. Bei der Aktivierung arbeitet man vorteilhaft in Gegenwart von 0 bis 8 Vol.-% Sauerstoff. Es können auch reduzierende Gase wie Wasserstoff oder Ethen enthalten sein.

Die Temperaturdifferenz über die Bandbreite kann hier sehr gut reguliert werden; sie soll im allgemeinen nicht mehr als 10, insbesondere nicht mehr als 5°C betragen.

Die erhaltenen Katalysatoren weisen einen sehr geringen Glühverlust von <1,5 Gew.-%, insbesondere <0,5 Gew.-% auf.

Der Silbergehalt der Katalysatoren beträgt 5 bis 25, insbesondere 9 bis 21 Gew.-%. Das Trägermaterial, das zur Herstellung der Katalysatoren verwendet wird, kann in Form von Kugeln, Ringen, Pellets oder solcher Körper vorliegen, die einen möglichst geringen Druckabfall im Reaktionsrohr verursachen. Bevorzugt werden Träger, die überwiegend aus $\alpha$-Aluminiumoxid bestehen, eingesetzt. Sie können bis zu 20 Gew.-% Siliciumdioxid enthalten. Die scheinbare Porosität liegt zwischen 35 und 65, insbesondere zwischen 40 und 60, vorteilhaft bei 55%. Der Porendurchmesser liegt z. B. zu 35—90% bei 1—100 μ und zu 60% bei 100—500 μ.

Die spezifische Oberfläche liegt vorteilhaft unter 10 m²/g, insbesondere unter 1 m²/g. Der Durchmesser der einzelnen Träger liegt vorteilhaft im Bereich von 5 bis 10 mm.

Bei Verwendung dieser Katalysatoren werden übliche Oxidationsbedingungen gewählt: Reaktionstemperaturen von 200 bis 300°C, Reaktionsdrücke zwischen 5 und 30 bar. Das Einsatzgasgemisch besteht aus 0,5 bis 40 Vol.-% Ethylen, 0 bis 60 Vol.-% Methan, 1 bis 15 Vol.-% Sauerstoff und als Rest andere inerte Bestandteile wie Stickstoff, Kohlendioxid oder Argon. Technisch wird dieses Gasgemisch in einem Kreisprozeß über den Kontakt geleitet, gebildetes Ethylenoxid abgetrennt, Kohlendioxid teilweise entfernt und nach Zusatz der Einsatzstoffe erneut über den Kontakt geleitet.

Die Herstellung der Silberkatalysatoren nach der vorliegenden Erfindung sowie die Verwendung dieser Katalysatoren für die Herstellung von Ethylenoxid wird in den nachfolgenden Beispielen im einzelnen beschrieben.

Beispiel 1

1. Herstellung des Katalysators

8000 g Silbernitrat (47 Mol) werden in 200 Liter vollentsalztem Wasser gelöst. Zur Fällung des Silberoxids wird eine Lösung von 1950 g Natriumhydroxid in 6 Liter vollentsalztem Wasser bei Raumtemperatur langsam unter Rühren zugegeben, so daß die Temperatur nicht über 25°C ansteigt. Das ausgefällte Silberoxid wird abgenuscht und mit vollentsalztem Wasser langsam gewaschen, bis das ablaufende Waschwasser salzfrei abläuft. Vom feuchten Silberoxid-Niederschlag werden 50% in

3

0 017 725

das Silberlactat durch Einsatz der stöchiometrischen Menge Milchsäure überführt. Den Rest des Silberoxids gibt man der wäßrigen Silberlactat-Lösung zu.

Anschließend wird diese Mischung mit 40 kg Katalysatorträger in einer heiz- und evakuierbaren Dragiertrommel zusammengebracht. Bei einer Temperatur von 85 bis 95°C und einem Druck von etwa 105 mbar wird in der Dragiertrommel, bei einer Drehzahl von etwa 10 UpM, die Silberoxid-Silberlactat-Mischung in und auf den Träger gebracht. Der Druck wird pro Minute etwa einmal durch kurzzeitiges Belüften auf 130 bis 160 mbar erhöht. Nach etwa 30 Minuten sind 85% der Wassermenge abgedampft.

Auf diese Weise produzierter Kontakt wird in ausreichender Menge im Stickstoffstrom bei 125°C in einer Siebbandmaschine vorgetrocknet. Das Band besteht aus einem geflochtenen V4A-Draht von 1,10 m Breite, hat eine Länge von 4,25 m × 2, eine Bandgeschwindigkeit von 6,77 cm/min. Daraus errechnet sich für den mit einer Schichtstärke von ca. 2 × Korndurchmesser aufgebrachten Kontakt eine Verweilzeit von etwa 90 min. Das Band ist mit Dampfregistern von oben und unten beheizt.

Nach der beschriebenen Methode wird in den Katalysator in gleicher Weise eine weitere Schicht aktiven Silbers eingebracht. Zu der Silberoxid-Silberlactat-Mischung werden eine Lösung von 6,7 g Cäsiumnitrat in Wasser und anschließend in die Dragiertrommel 30 g Bariumperoxid zugegeben.

## 2. Die Aktivierung

Der so vorgetrocknete Katalysator wird in einer zweiten, analog konstruierten Siebbandmaschine aktiviert. Die Bandgeschwindigkeit liegt bei 5,64 cm/min; daraus errechnet sich eine Verweilzeit von 75 min. Das Band wird ebenfalls mit Dampfregistern von oben und unten beheizt. Die höchste Temperatur betrug 210°C, die Temperaturdifferenz über die Breite des Bandes 5°C. Über das Band wurde im Gegenstrom 10 m³/h Stickstoff mit einem Sauerstoffgehalt von 5 Vol.-% geführt. Die Analyse des fertiggestellten Katalysators zeigt, daß dieser 19,45 Gew.-% Silber, 481 ppm Barium und 91 ppm Cäsium enthält. In dem so gewonnenen Katalysator wurde noch ein Glühverlust von 0,4 Gew.-% festgestellt, der zur Hauptsache aus Wasser bestand. Das Silber auf dem Katalysatorträger haftet so fest, daß der Katalysator beim Umfüllen und Schütteln nicht staubt.

## 3. Test des Katalysators

Der Katalysator wird in einer Apparatur geprüft, die aus einem Reaktionsrohr von 6000 mm Länge und 26 mm Durchmesser aus rostfreiem Stahl besteht. Das Reaktionsrohr ist von einem Mantel umgeben, der Wasser bzw. Wasserdampf zum Abführen der Reaktionswärme enthält.

Das Reaktionsrohr wird mit 2,7 Liter = 2,8 kg endformiertem Katalysator gefüllt.

Eine Gasmischung, bestehend aus

| | |
|---|---|
| 25 Vol.-% | $C_2H_4$ |
| 49 Vol.-% | $CH_4$ |
| 6,8 Vol.-% | $O_2$ |
| 4,5 Vol.-% | $CO_2$ |
| 0,2 Vol.-% | $C_2H_6$ |
| Rest | $Ar + N_2$ |

wird mit einem Durchsatz von 20 Nm³/h über den Katalysator geleitet. Der Druck im Reaktor beträgt 19,8 bar-Überdruck und die Temperatur im Dampfraum des Kühlmantels liegt bei 247°C. Der Gesamtchlorgehalt im Einsatzgas liegt bei 8 mg Cl/Nm³, was durch eine spezielle Dosierung eines Moderators erreicht wird.

Nach beendeter Einlaufphase des frisch eingefüllten Kontaktes werden ein Sauerstoffumsatz von 41 Mol.-%, eine Selektivität von 79,1 Mol.-% und eine Ethylenoxidkonzentration im Reaktorausgang von 2,13 Vol.-% gefunden.

## Beispiel 2

Unter Verwendung des im Beispiel 1 eingesetzten Trägermaterials und des dort beschriebenen Verfahrens werden eine Reihe von Katalysatoren mit unterschiedlichem Bariumgehalt aber in etwa konstantem Cäsiumgehalt hergestellt und in analoger Weise, wie im Beispiel 1, untersucht. Die Katalysatorzusammensetzungen und deren Eigenschaften sind in Tabelle 1 zusammengefaßt.

## Beispiel 3

Unter Verwendung des im Beispiel 1 beschriebenen Verfahrens werden eine Reihe von Katalysatoren mit verschiedenen Cäsiumgehalten aber konstantem Bariumgehalt hergestellt.

4

Die fertigen Katalysatoren wurden in analoger Weise untersucht. Die Ergebnisse sind in Tabelle 2 zusammengefaßt.

## Beispiel 4

Wie im Beispiel 1 beschrieben, wurde unter Verwendung des gleichen Trägermaterials, jedoch unter Verwendung verschiedener Alkalimetallzusätze, eine Reihe von Katalysatoren hergestellt und in analoger Weise, wie im Beispiel 1 beschrieben, untersucht. Die Ergebnisse dieser Untersuchungen sind in der Tabelle 3 zusammengefaßt.

## Beispiel 5

Nach dem gleichen Verfahren, wie im Beispiel 1 beschrieben, wurde eine Reihe von Katalysatoren hergestellt, wobei jeweils verschiedene Trägermaterialien eingesetzt wurden; der Zusatz von Bariumperoxid und Cäsiumnitrat als Promotoren wurde beibehalten.

Der Test dieser Katalysatoren wurde in analoger Weise wie im Beispiel 1 beschrieben, durchgeführt; die Ergebnisse der Untersuchungen sind in Tabelle 4 zusammengefaßt.

## Vergleichsbeispiel

Ein Katalysator, welcher nach dem Imprägnierverfahren hergestellt worden war und bei welchem mit der Silbersalzlösung 270 ppm Barium und 53 ppm Cäsium nach der Aktivierung eingebracht worden waren, und der nicht in dünner bewegter Schicht getrocknet und aktiviert worden war, wurde bei einem Silbergehalt von 14,1 Gew.-% unter den Bedingungen getestet, wie dies im Beispiel 1, Pkt. 3: Test des Katalysators beschrieben ist.

Nach beendeter Einlaufphase des frisch eingefüllten Kontaktes wurden bei einer Dampfraumtemperatur von 258°C, einem Gesamtchlorgehalt von 6,3 mg Cl/Nm$^3$ ein Sauerstoffumsatz von 44,1 Mol-%, eine Ethylenoxidkonzentration von 1,91 Mol-% im Reaktorausgang bei einer Selektivität von 73,7 Mol-% gefunden.

Tabelle 1

| Versuch | Ag-Gehalt | Barium | Cäsium | Akti-vierung | Dampf-raum-temp. | O$_2$-Ums. | EO | Selek. |
|---------|-----------|--------|--------|--------------|------------------|------------|------|--------|
|         | %         | ppm    | ppm    |              | °C               | Mol-%      | Vol.-% | Mol-% |
| 2a      | 19,8      | 97     | 85     | Siebband     | 254              | 37,2       | 1,97 | 79,3   |
| 2b      | 19,7      | 223    | 89     | Siebband     | 251              | 40,2       | 2,07 | 78,5   |
| 2c      | 19,5      | 470    | 81     | Siebband     | 248              | 40,3       | 2,11 | 79,0   |

Tabelle 2

| Versuch | Ag-Gehalt | Barium | Cäsium | Akti-vierung | Dampf-raum-temp. | O$_2$-Ums. | EO | Selek. |
|---------|-----------|--------|--------|--------------|------------------|------------|------|--------|
|         | %         | ppm    | ppm    |              | °C               | Mol-%      | Vol.-% | Mol-% |
| 3a      | 20,2      | 465    | 91     | Siebband     | 248              |            | 2,03 | 78,9   |
| 3b      | 19,6      | 477    | 47     | Siebband     | 245              |            | 2,00 | 79,2   |
| 3c      | 19,7      | 480    | 173    | Siebband     | 253              |            | 2,15 | 78,1   |

Tabelle 3

| Versuch | Ag-Gehalt | Barium | Alkali | Akti-vierung | Dampf-raum-temp. | O₂-Ums. | EO | Selek. |
|---------|-----------|--------|--------|--------------|------------------|---------|-----|--------|
| | % | ppm | ppm | | °C | Mol-% | Vol.-% | Mol-% |
| 4a | 19,5 | 471 | 90 | Siebband | 248 | 40,3 | 2,11 | 79,0 |
| 4b | 19,4 | 465 | 95 | Siebband | 253 | 41,2 | 2,03 | 77,3 |

Tabelle 4

| Versuch | Ag-Gehalt | Barium | Cäsium | Dampf-raum-temp. | O₂-Ums. | EO | Selek. |
|---------|-----------|--------|--------|------------------|---------|-----|--------|
| | % | ppm | ppm | °C | Mol-% | Vol.-% | Mol-% |
| 5a | 19,5 | 471 | 95 | 248 | 40,3 | 2,11 | 79,0 |
| 5b | 18,8 | 451 | 91 | 249 | 40,1 | 2,05 | 78,3 |

**Patentanspruch**

Verfahren zur Herstellung von Silberkatalysatoren, enthaltend Erdalkali- und Alkalimetalle als Promotoren für die Herstellung von Ethylenoxid durch Oxidation von Ethylen mit Sauerstoff oder Sauerstoff-enthaltenden Gasen durch die Kombination folgender Merkmale:
Aufbringen einer Suspension, die besteht aus Silberoxid und einer wäßrigen Lösung eines organischen Silbersalzes auf einen porösen Träger, wobei das molare Verhältnis Silber in Silberoxid zu Silber im Silbersalz 35 bis 65% beträgt und während des Aufziehens periodisch verminderter Druck angewendet wird,
Zwischentrocknen in dünner bewegter Schicht unter Inertgasatmosphäre bei Temperaturen von 50 bis 150° C,
Aufbringen einer weiteren Schicht der silberhaltigen Suspension in der beschriebenen Weise, wobei diese Suspension zusätzlich Barium- und Cäsium- und/oder Rubidiumverbindungen in solchen Mengen enthält, daß im fertigen Katalysator 0,01 bis 0,25 Gewichtsprozent Barium und 0,001 bis 0,05 Gewichtsprozent Cäsium und/oder Rubidium, gerechnet als Metalle, enthalten sind, und schließlich Erhitzen in dünner bewegter Schicht auf Temperaturen von 160 bis 270° C.

**Claim**

A process for the manufacture of a silver catalyst, containing an alkaline earth metal and an alkali metal as promoters, for the manufacture of ethylene oxide by oxidation of ethylene with oxygen or an oxygen-containing gas, comprising the combination of the following steps:
application of a suspension, consisting of silver oxide and an aqueous solution of an organic silver salt, onto a porous carrier, the molar proportion of silver in the silver oxide to silver in the silver salt being from 35 to 65%, and periodically reduced pressure being applied during the absorption,
intermediate drying in a thin moving layer in an inert gas atmosphere, at a temperature from 50 to 150° C,
application of a further layer of the silver-containing suspension in the specified manner, the suspension in this case containing in addition barium and cesium and/or rubidium compounds in such proportions that the finished catalyst contains from 0.01 to 0.25 per cent by weight of barium and from 0.001 to 0.05 per cent by weight of cesium and/or rubidium, calculated as metals, and finally heating in a thin moving layer to a temperature of 160 to 270° C.

**Revendication**

Procédé pour la préparation de catalyseurs à base d'argent, renfermant des métaux alcalino-terreux et alcalins comme promoteurs pour la préparation d'oxyde d'éthylène par oxydation de l'éthylène avec l'oxygène ou des gaz contenant de l'oxygène par la combinaison des caractéristiques suivantes:

introduction d'une suspension, constituée d'oxyde d'argent et d'une solution aqueuse d'un sel organique d'argent sur un support poreux, le rapport molaire argent dans l'oxyde d'argent étant compris entre 35 et 65% et une pression réduite étant appliquée périodiquement pendant l'absorption, séchage intermédiaire dans une mince couche en mouvement sous atmosphère de gaz inerte à des températures comprises entre 50 et 150°C,

introduction d'une autre couche de la suspension contenant de l'argent selon la manière décrite, cette suspension contenant en plus des combinaisons de baryum et de césium et/ou de rubidium dans des quantités telles que le catalyseur préparé contienne de 0,01 à 0,25 pour-cent en poids de baryum et de 0,001 à 0,05 pour-cent en poids de césium et/ou de rubidium, calculés en métaux, et finalement chauffage dans une mince couche en mouvement à des températures comprises entre 160 et 270°C.